**Europäisches Patentamt**

**European Patent Office**

**Office européen des brevets**

(19)

(11) Veröffentlichungsnummer: **0 235 139**
**B1**

(12) # EUROPÄISCHE PATENTSCHRIFT

(45) Veröffentlichungstag der Patentschrift:
03.05.89

(51) Int. Cl.⁴: **A 61 M 5/32**, A 61 M 5/34

(21) Anmeldenummer: 85905732.5

(22) Anmeldetag: **20.11.85**

(86) Internationale Anmeldenummer:
PCT/AT 85/00048

(87) Internationale Veröffentlichungsnummer:
WO 86/03126 (05.06.86 Gazette 86/12)

(54) INJEKTIONSSPRITZE.

(30) Priorität: **21.11.84 AT 3688/84**

(43) Veröffentlichungstag der Anmeldung:
**09.09.87 Patentblatt 87/37**

(45) Bekanntmachung des Hinweises auf die Patenterteilung:
**03.05.89 Patentblatt 89/18**

(84) Benannte Vertragsstaaten:
**AT BE FR**

(56) Entgegenhaltungen:
**AU-B- 22 732**
**DE-A- 3 317 675**
**FR-A- 399 155**
**FR-A- 2 186 270**
**FR-A- 2 439 021**
**FR-A- 2 507 897**
**US-A- 3 073 307**
**US-A- 3 096 763**

(73) Patentinhaber: **PICKHARD, Ewald,**
**Redtenbachergasse 15, A-1160 Wien (AT)**

(72) Erfinder: **PICKHARD, Ewald, Redtenbachergasse 15,**
**A-1160 Wien (AT)**

(74) Vertreter: **Wolke, Heidemarie, Dr., Stadtplatz 7,**
**A-4400 Steyr (AT)**

## Beschreibung

Die Erfindung bezieht sich auf eine Injektionsspritze, wie sie im Oberbegriff des Patentanspruches 1 beschrieben ist.

Bei derartigen Injektionsspritzen für die Einmalverwendung ist es bereits bekannt, zum Schutz der Spritzennadel abziehbare Kappen vorzusehen, welche die Nadel beim Transport schützen und die Gefahr einer Verletzung verringern sollen. In der Regel wird die Injektionsspritze erst unmittelbar vor der Verwendung mit einer derartigen Spritzennadel zusammengesetzt.

Es ist bereits eine Injektionsspritze bekannt — gemäss US-A-3 073 307 —, die einen Spritzenkörper aufweist, in welchem ein Nadelträger eingesetzt ist. In den Nadelträger ist die Spritzennadel eingeschäumt bzw. eingeklebt. Mit dem Nadelträger ist weiters eine Haltevorrichtung kraft- und bzw. oder formschlüssig verbunden, die mit einem Kupplungsteil des Spritzenkörpers, beispielsweise einem Luer-Kegel formschlüssig kuppelbar ist. Die Spritzennadel ist während der Herstellung der Injektionsspritze sowie während des Transportes von einer Nadelschutzkappe umhüllt, die sich bis in den Bereich des Nadelträgers erstreckt. Zwischen dem Nadelträger und der Nadelschutzkappe ist eine Öffnungsvorrichtung angeordnet, die durch eine Schwächungslinie gebildet ist. Dadurch wird die Manipulation der Spritzennadeln während der Montage und dem Zusammenbau der Injektionsspritze vor allem beim Befüller, bei welchem das Medikament in den Spritzenkörper eingefüllt und danach die Spritzennadel aufgesetzt wird, vereinfacht. Es kann jedoch nicht verhindert werden, dass bei bestimmten Medikamenten diese im Ausgangsbereich zur Spritzennadel bzw. im Spritzennadelkanal kristallisierten und dadurch die Injektionsspritze unbrauchbar wird.

Aus der US-A-3 096 763 war es weiters bereits bekannt, anstelle des Luer-Konus eine kraft- und formschlüssige Kupplungsvorrichtung zwischen dem Spritzenkörper und dem Nadelträger vorzusehen. Der Spritzenkörper ist in diesem Fall in seinem dem Nadelträger zugewandten Endbereich mit einer umlaufenden Nut versehen, in die mit Vorsprüngen versehene und aus elastischem Werkstoff, beispielsweise biegsamem Kunststoff, hergestellte Schnappfinger, die mit dem Nadelträger kraft- und formschlüssig verbunden sind, einschnappen. Zur besseren Führung des Nadelträgers während des Aufschnappens der Haltevorrichtung auf dem Spritzenkörper ist der Nadelträger auf der von der Spritzennadel abgewendeten Seite mit einem Führungsteil versehen. Dieser Führungsteil weist eine Aussenform auf, die im wesentlichen der Innenform einer Auslassöffnung des Spritzenkörpers angepasst ist und beim Aufschieben des Nadelträgers auf den Spritzenkörper eine Führung des Nadelträgers bewirkt, so dass das Einschnappen der Schnappfinger erleichtert wird. Dadurch konnte zwar die Montage des Nadelträgers am Spritzenkörper vereinfacht werden, ein Austreten des Medikamentes in den Spritzennadelkanal während des Transportes konnte dadurch aber ebenfalls nicht verhindert werden.

Bei Verpackungen von Nahrungs- und Genussmitteln ebenso wie zu verabreichenden Pharmazeutika ist es bereits bekannt, einen Garantieverschluss vorzusehen, welcher die Feststellung ermöglicht, ob eine Packung bereits angebrochen bzw. geöffnet wurde oder nicht. Es ist weiters bereits bekannt, im Fall von Spritzennadeln diese in einem Kunststoffbehälter, beispielsweise einem Kunststoffsack, steril verpackt und verschweisst zu lagern.

Die Erfindung zielt nun darauf ab, eine Injektionsspritze mit bereits angesetzter Nadel zu schaffen, welche vom Zeitpunkt der Füllung bis zu ihrer Verwendung ohne zusätzliche Schutzmassnahmen steril verpackt ist und nur in einer Weise geöffnet werden kann, welche nachträglich die Tatsache, dass die Spritze bereits geöffnet wurde, klar erkennen lässt. Gleichzeitig soll der Verbleib des Medikamentes im Spritzenkörper bis zum Gebrauch der Injektionsspritze möglich sein.

Diese Aufgabe der Erfindung wird durch die Merkmale im Kennzeichenteil des Patentanspruches 1 gelöst. Durch die Anordnung einer Verschlussvorrichtung wird erreicht, dass auch bei unterschiedlichen Druck- und Temperaturverhältnissen die im Spritzenkörper enthaltene Injektionsflüssigkeit nicht durch die Spritzennadel, beispielsweise in den Hohlraum in der Nadelschutzkappe austreten und gegebenenfalls dort kristallisieren kann. Damit können vor allem die grossen Druck- und Temperaturunterschiede, welchen Injektionsspritzen, z. B. während eines Transportes mit Flugzeugen, ausgesetzt sind, nicht mehr ein Austreten der Medikamente bewirken. Gleichzeitig kann aber die Nadelschutzkappe nunmehr auch dazu verwendet werden, um den Garantieverschluss für die weitere, durch eine Membran gebildete Verschlussvorrichtung zu bilden. Dadurch wird sichergestellt, dass erst nachdem der Originalitäts- bzw. Garantieverschluss geöffnet wurde, auch die Verbindung zwischen Spritzennadelkanal und Spritzenkörper hergestellt werden kann und dadurch das Medikament erst unmittelbar vor Gebrauch der Injektionsspritze in den Spritzenkanal eintreten kann. Dadurch wird die Sterilität gewahrt, aber auch gleichzeitig eine etwaige Kristallisation des Medikamentes im Spritzenkanal zuverlässig vermieden. Überdies wird ein Luftzutritt zum Medikament in die meist aus Glas bestehende Spritzenkörper zuverlässig unterbunden.

Von Vorteil ist eine Ausbildung gemäss Patentanspruch 2. Durch die Ausbildung und Anordnung des Öffnungsorgans kann mit der Nadelschutzkappe gleichzeitig die Sterilität des Spritzennadelkanals und der diesem zugeordneten Öffnungsvorrichtung und der Spritzennadel erreicht werden.

Vorteilhaft ist auch eine Weiterbildung der Injektionsspritze nach Patentanspruch 3. Durch Verwendung zweier gegeneinander verstellbarer Teile in Verbindung mit einer Membran ist sichergestellt, dass auch beim Öffnen der Membran die Sterilität der Injektionsspritze gewahrt ist.

Vorteilhaft ist auch eine Ausbildung der Injektionsspritze gemäss Patentanspruch 4. Durch die Verwendung konischer Ansatzflächen und der Ausbildung der Oberfläche des Führungsteils als Dichtfläche kann eine zuverlässige Abdichtung zwischen Nadelträger und Auslassöffnung sichergestellt werden.

Eine vorteilhafte Weiterbildung der Injektionsspritze ist auch im Patentanspruch 5 enthalten. Durch die Anordnung einer Dichtung zwischen dem Ringflansch und der diesem zugewandten Stirnseite des Ansatzstückes können Toleranzen im Fertigungsprozess ohne nachteilige Beeinflussung der Dichtheit und Sterilität der Injektionsspritzen ausgeglichen werden.

Durch eine Ausbildung gemäss Patentanspruch 6 wird erreicht, dass bei der Relativbewegung zwischen dem Nadelträger und der Haltevorrichtung das Eindringen von Bakterien in Richtung des Spritzenkörpers verhindert ist.

Eine weitere Ausführungsform ist im Patentanspruch 7 beschrieben. Dadurch wird ein fester Sitz der Haltevorrichtung am Ansatzstück sichergestellt.

Von Vorteil ist auch eine Ausführungsform gemäss Patentanspruch 8, da dadurch die Nadel so lange geschützt und steril gehalten ist, bis die Verschlussvorrichtung geöffnet ist und erst danach knapp vor dem Einstechen der Injektionsnadel die Nadelschutzkappe zur Gänze entfernt werden muss.

Zum besseren Verständnis der Erfindung wird diese im folgenden anhand der in den Zeichnungen dargestellten Ausführungsbeispiele näher erläutert.

Es zeigen:

Fig. 1 eine Injektionsspritze mit angesetzter Nadelschutzkappe axial geschnitten;

Fig. 2 eine Teilansicht der Injektionsspritze nach Fig. 1 vor der Verwendung;

Fig. 3 eine verkleinerte Teilansicht der Injektionsspritze nach Fig. 2 nach Abnahme des vorderen Teiles der Nadelschutzkappe;

Fig. 4 eine erfindungsgemässe Ausführung einer Injektionsspritze mit einer im Zuge des Spritzenkanals angeordneten Verschlussvorrichtung und einer dieser zugeordneten Öffnungsvorrichtung, die innerhalb der Nadelschutzkappe angeordnet ist;

Fig. 5 eine Ausführungsvariante der Öffnungsvorrichtung für eine Injektionsspritze nach Fig. 4 mit zwei im Bereich der Nadelschutzkappe angeordneten Öffnungsvorrichtungen;

Fig. 6 eine andere Ausführungsform einer innerhalb der Nadelschutzkappe angeordneten Verschlussvorrichtung für den Spritzkanal, wobei nur ein Teil der Injektionsspritze axial geschnitten dargestellt ist;

Fig. 7 einen Teil der Injektionsspritze nach Fig. 6 in Draufsicht bei abgetrennter Nadelschutzkappe und teilweise geschnitten;

Fig. 8 eine Ausführungsvariante einer Verschlussvorrichtung für eine erfindungsgemässe Injektionsspritze, von der nur ein Teil axial geschnitten dargestellt ist.

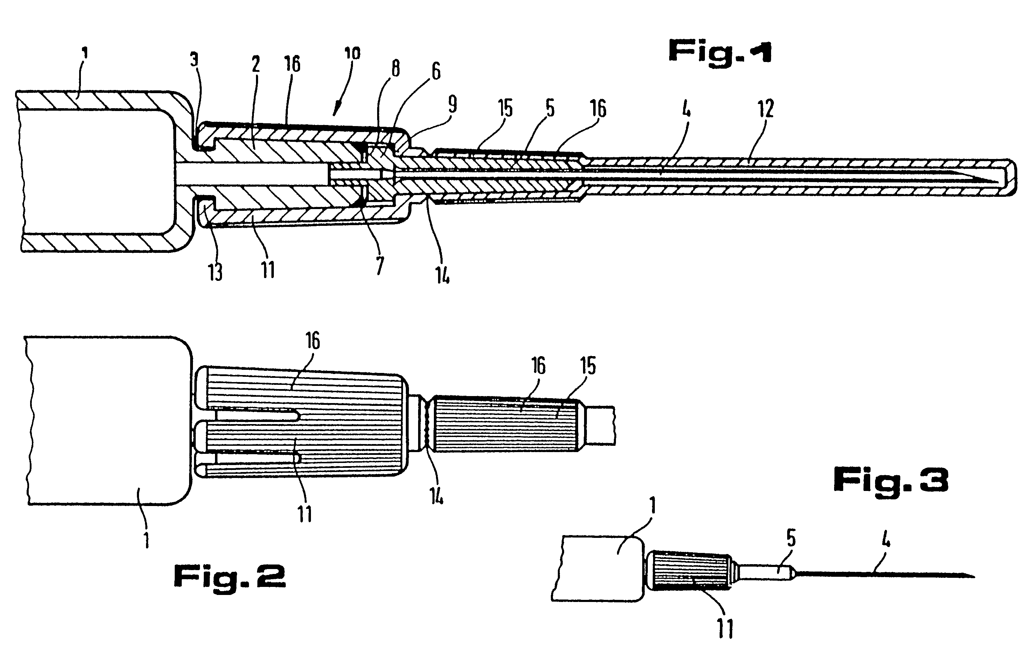

In Fig. 1 ist der das Injektionsmedium aufnehmende Spritzenkörper mit 1 bezeichnet. Der Spritzenkörper weist ein konisches Ansatzstück 2 auf, welches einstückig mit dem Spritzenkörper 1 ausgebildet ist. An der Übergangsstelle zwischen dem Ansatzstück 2 und dem Spritzenkörper 1 ist als nutförmige Vertiefung eine Ringnut 3 angeordnet. Eine Spritzennadel 4 ist mit einem Nadelträger 5 verbunden, bevorzugt an diese angespritzt. Der Nadelträger 5 weist einen Ringflansch 6 auf, welcher unter Zwischenschaltung einer Dichtung 7 an eine dem Nadelträger 5 zugewandte Stirnfläche 8 des Ansatzstückes 2 anpressbar ist. Der Ringflansch 6 ist an seiner der Spritzennadel 4 zugewendeten Stirnfläche 9 mit einer zweiteiligen Schutzkappe 10 bestehend aus Haltevorrichtung 11 und Nadelschutzkappe 12 verbunden, wobei die Verbindung durch Ultraschallverschweissung erzielt wurde.

Beim Zusammenbau der Injektionsspritze zur gleichzeitigen Erzielung eines sterilen Abschlusses wird die aus Nadelträger 5, Spritzennadel 4 und Schutzkappe 10 bestehende Einheit auf die konische Mantelfläche des Ansatzstückes 2 aufgeschoben, wobei einwärts springende Klauen 13 der als Basisteil dienenden Haltevorrichtung 11 der Schutzkappe 10 in die Ringnut 3 eintauchen.

Die Schutzkappe 10 weist neben dem Basisteil zur kraftschlüssigen Verbindung des Nadelträgers 5 mit dem Ansatzstück 2 weiters eine die Nadel übergreifende Nadelschutzkappe 12 auf, welche über eine Sollbruch- bzw. Abreissstelle bzw. Schwächungslinie 14 mit dem Basisteil in Verbindung steht.

In Fig. 1 ist ersichtlich, dass sowohl die als Basisteil dienende Haltevorrichtung 11 als auch ein den Nadelträger 5 übergreifender Teilbereich 15 der die Spritzennadel umgreifenden Nadelschutzkappe 12 in Längsrichtung verlaufende Rillen 16 aufweist. Auf diese Weise wird die Handhabbarkeit, insbesondere die Verdrehbarkeit der beiden Teile gegeneinander, zum Zwecke des Aufreissens an der Sollbruchstelle bzw. Schwächungslinie 14 verbessert.

Nach dem Durchtrennen der Sollbruchstellen, die beispielsweise von Stegen oder dgl. gebildet sein können, kann die die Nadel übergreifende Nadelschutzkappe 12 der Schutzkappe 10 abgezogen werden und die Injektionsspritze kann unmittelbar verwendet werden. Eine derartige Injektionsspritze mit abgezogener Nadelkappe ist in Fig. 3 dargestellt.

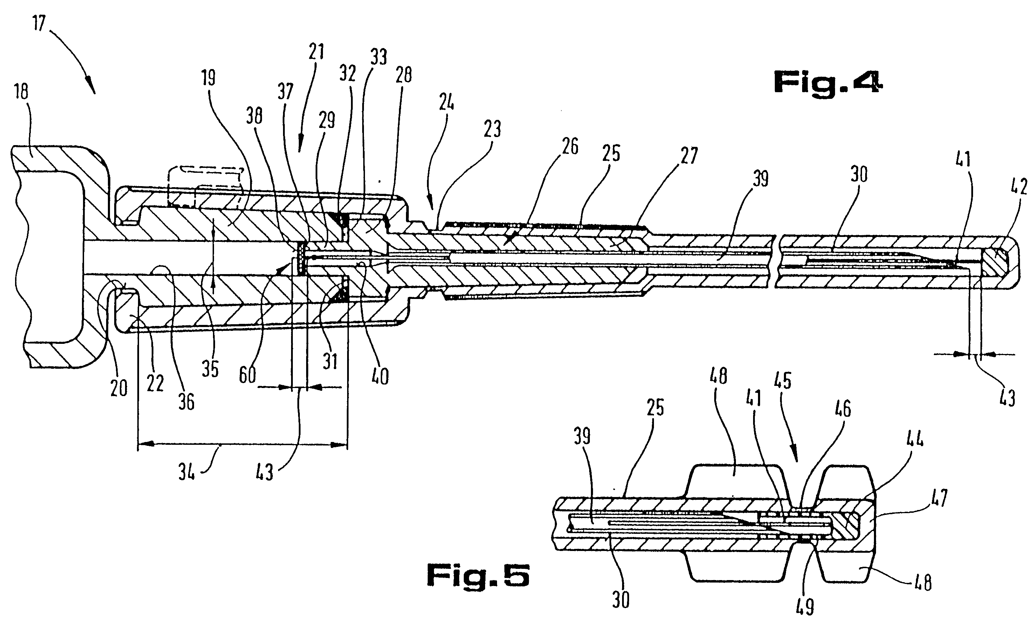

In Fig. 4 ist von einer Injektionsspritze 17 ein Spritzenkörper 18, der in den meisten Fällen aus Glas besteht, gezeigt, an dem einstückig ein Ansatzstück 19 angeformt ist, wobei zwischen dem Spritzenkörper 18 und dem Ansatzstück 19 als Kupplungsteil eine nutförmige Vertiefung 20 angeordnet ist. Das Ansatzstück 19 wird von einer Haltevorrichtung 21 bzw. dessen Kegelmantel übergriffen, der in dem dem Spritzenkörper 18 zugewandten Ende mit klauenförmigen Vorsprüngen 22 in die nutförmige Vertiefung 20 zwischen Ansatzstück 19 und Spritzenkörper 18 eingreift. Dieser Vorsprung kann durch einen umlaufenden Ringwulst oder durch mehrere jeweils nur Segmente des Kegelmantels umfassende Klauen gebildet sein. Die Haltevorrichtung 21 ist über eine durch eine Schwächungslinie 23 gebildete Öffnungsvorrichtung 24 mit einer Nadelschutzkappe 25 einstückig verbunden. Die Haltevorrichtung 21 ist weiters, beispielsweise durch einen Ultraschallschweissvorgang, mit einem Nadelträger 26 kraft- und formschlüssig verbunden. Dieser Nadelträger 26 weist einen Stützkörper 27, der bevorzugt wie im vorliegenden Ausführungsbeispiel kegelförmig ausgebildet sein kann, einen an diesen anschliessenden Ringflansch 28 und in Richtung des Spritzenkörpers 18 einen Führungsteil 29 auf. Dieser Nadelträger 26 ist bevorzugt aus einem spritzfähigen Kunststoff hergestellt, wobei die Spritzennadel 30 in den Nadelträger 26 eingespritzt ist. Eine Stirnseite 31 des Ringflansches 28 liegt bevorzugt über eine Dichtung 32, beispielsweise einen O-Ring, an einer dem Nadelträger zugewandten Stirnseite 33 des Ansatzstückes 19 an. Eine Distanz 34 zwischen einer Flanke der nutförmigen Vertiefung 20 und der Stirnseite 31 entspricht im wesentlichen einer Distanz zwischen derselben Seitenflanke der nutförmigen Vertiefung und der Stirnseite 33 des Ansatzstückes 19. Vor allem dann, wenn keine Dichtung 32 vorgesehen ist, ist im entspannten Zustand des Vorsprunges 22 die Distanz 34 geringfügig kleiner als die entsprechende Distanz des Ansatzstückes 19, um eine sichere Anlage der Stirnseite 31 an der Stirnseite 33 des Ansatzstückes 19 und somit eine absolute Dichtheit zu ermöglichen. Der Führungsteil 29 weist beim dargestellten Ausführungsbeispiel in etwa einen gleichen Durchmesser 35 auf wie eine Auslassöffnung 36 des Ansatzstückes 19. Eine Länge des Führungsteiles 29 in Richtung der Auslassöffnung 36 ist so bemessen, dass sie grösser ist als eine in derselben Richtung vorgesehene Dicke des Vorsprunges 22, so dass während des Einschnappens der Vorsprünge 22 aus der in strichlierten Linien angedeuteten, aufgeweiteten in die in vollen Linien gezeichnete eingeschnappte Stellung eine zentrische Führung der Haltevorrichtung 21 mit dem Führungsteil 29 in der Auslassöffnung 36 gewährleistet ist. Dadurch wird ein zentrischer Sitz des Nadelträgers 26 und eine exakte Abdichtung zwischen Nadelträger 26 und Ansatzstück 19 erreicht.

Im Bereich einer dem Spritzenkörper 18 zugewandten Stirnseite 37 kann eine durch eine flüssigkeitsdichte Membran 38 gebildete Verschlussvorrichtung angeordnet sein. Zum Durchstossen dieser Membran und zum Herstellen einer Verbindung zwischen der Auslassöffnung 36 und einem Einspritzende der Spritzennadel 30 bzw. dem in der Spritzennadel angeordneten Spritzennadelkanal 39 und einem Spritzkanal 40 im Nadelträger 26 ist eine durch eine Nadel 41 gebildete Öffnungsvorrichtung angeordnet. Die Nadel 41 der Öffnungsvorrichtung erstreckt sich von dem in der Spitze der Spritzennadel 30 angeordneten Ende der Nadelschutzkappe 25 bis knapp vor die Membran 38. Wird nun die Nadelschutzkappe 25 durch Verdrehen gegenüber der Haltevorrichtung 21, wozu die beiden letztgenannten Teile, wie aus der Zeichnung ersichtlich, mit einer Riffelung — wie bereits in den Fig. 1 bis 3 gezeigt — oder mit Betätigungsflanschen versehen sein können, geöffnet und die Nadelschutzkappe 25 abgezogen, so kann mit einem am Nadelende angeordneten Betätigungsorgan 42 die Nadelspitze um eine Wegstrecke 43 vorgeschoben werden, so dass die Nadelspitze die Membran 38 durchstösst und eine Verbindung zwischen dem Spritzennadelkanal 39 und der Auslassöffnung 36 herstellt. Nach dem Durchstossen der Membran wird die Nadel 41 mit dem Betätigungsorgan 42 herausgezogen und die Injektionsspritze 17 ist bereit zum Einspritzen der Injektionslösung. Der hierzu im Spritzenkörper 18 vorgesehene Kolben und die Betätigungsvorrichtung hierfür sowie die Injektionslösung sind der besseren Übersichtlichkeit der Zeichnung wegen nicht dargestellt und es können hierzu alle aus dem Stand der Technik bekannten Ausführungsformen verwendet werden.

In Fig. 5 ist eine Ausführungsvariante eines Betätigungsorganes 44 sowie eine weitere Öffnungsvorrichtung 45 im Zuge der Nadelschutzkappe 25 gezeigt. Es werden für gleiche Teile dieselben Bezugsziffern wie in Fig. 4 verwendet. Auch der Ablauf des Durchstossens der Membran 38 ist beim vorliegenden Ausführungsbeispiel gleich wie zu Fig. 4 beschrieben.

Die Nadelschutzkappe 25 weist jedoch im Bereich des Betätigungsorganes 44 eine weitere Öffnungsvorrichtung 45 auf, die ebenfalls durch eine Schwächungslinie 46, beispielsweise durch Perforationen oder einen Aufreissring oder dgl. gebildet sein kann. Jedenfalls handelt es sich um eine Öffnungsvorrichtung, die nach einmaligem Öffnen nicht mehr so verschlossen werden kann, so dass auch von einem neutralen Betrachter sofort erkannt werden kann, ob die Öffnungsvorrichtung bereits geöffnet wurde oder nicht. Hierzu sind auf der Nadelschutzkappe 25 und auf dem weiteren Teil 47 der Nadelschutzkappe Flügel 48 angeordnet, um ein sicheres Öffnen bzw. Aufbrechen im Bereich der Schwächungslinie 46 und eine Trennung des Teiles 47 von der Nadelschutzkappe 25 zu ermöglichen. Das Betätigungsorgan 44 umfasst zusätzlich zu der in Fig. 4 gezeigten Ausführungsform eine sich in Richtung der Spritzennadel 30 erstreckende rohrförmige Schutzhülle 49, deren Innendurchmesser grösser ist als der Aussendurchmesser der Spritzennadel 30 und deren Aussendurchmesser geringfügig kleiner ist als der Innendurchmesser der Nadelschutzkappe 25. Nach dem Abtrennen des Teiles 47 von der Nadelschutzkappe 25 wird dadurch gewährleistet, dass durch die Schutzhülle 49 ein steriler Abschluss der Spritzennadel 30 gegenüber der Öffnungsvorrichtung bzw. dem Betätigungsorgan 44 gegeben ist, und es wird sichergestellt, dass auch beim Verschieben des Betätigungsorgans 44 um die Wegstrecke 43, wie in Fig. 4 erläutert, keine von der Bedienungsperson der Injektionsspritze berührten oder nicht sterilisierten Teile während des Durchstechens der Membran 38 mit der Spritzennadel bzw. dem Inneren der Nadelschutzkappe 25 in Berührung kommen. Erst nachdem die Nadelschutzkappe 25 abgetrennt wurde bzw. die Nadel 41 aus dem Spritzennadelkanal 39 herausgezogen wurde, ist diese zum Einspritzen der Injektionslösung bereit.

Dadurch wird neben einem einfachen Aufbau einer Nadelschutzvorrichtung bzw. einer Schutzkappe für nur einmal zu verwendende Injektionsspritzen eine einwandfreie Sterilität bis zum Gebrauch der Injektionsspritze erreicht und es wird überdies das Austreten von Injektionslösung bzw. das Eintreten von Luft in die Injektionsspritze und eventuellen Kristallisierungen der Injektionslösung hinreichend vorgebeugt.

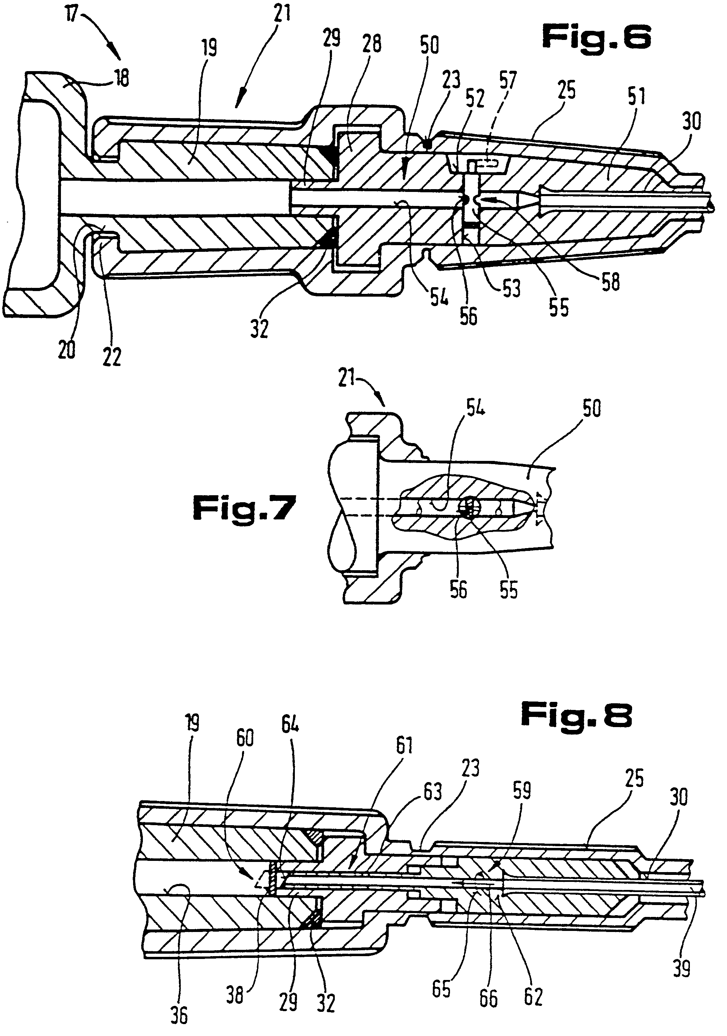

In Fig. 6 ist eine andere Ausführungsvariante einer Verschlussvorrichtung bzw. eines dieser zugeordneten Öffnungsorganes dargestellt. Der Aufbau des Spritzenkörpers bzw. der Haltevorrichtung und des Ansatzstückes entspricht demjenigen in Fig. 4, so dass für gleiche Teile wiederum gleiche Bezugszeichen verwendet werden.

Ebenso weist ein Nadelträger 50 einen Ringflansch 28 und einen Führungsteil 29 in der ebenfalls vorbeschriebenen Form auf. Auch die Anordnung der Nadelschutzkappe 25 und die durch eine Schwächungslinie 23 gebildete Öffnungsvorrichtung 24 entspricht der in Fig. 4 beschriebenen Ausführungsform. Lediglich ein Stützkörper 51 weist einen gegenüber der Fig. 4 grösseren Durchmesser auf. Überdies ist die Spritzennadel 30 in einem grösseren Abstand vom Ansatzstück 19 in den Nadelträger 50 eingespritzt, als dies bei der Ausführungsform nach Fig. 4 der Fall ist. Des weiteren weist der Stützkörper 51 eine Vertiefung 52 auf.

Senkrecht zu einer ebenen Grundfläche dieser Vertiefung 52 ist eine Bohrung 53 angeordnet, die zentrisch aber senkrecht zu einem Spritzkanal 54 des Nadelträgers 50 verläuft. In diese Bohrung 53 ist ein Ventilstift 55 eingesetzt.

In Fig. 7 ist ersichtlich, dass der Ventilstift 55 in der geschlossenen Stellung senkrecht zur Längsrichtung des Spritzkanals 54 des Nadelträgers 50 Abflachungen 56 aufweist. In den in vollen Linien in Fig. 6 und 7 gezeichneten Stellungen ist somit ein Durchgang einer Injektionslösung von dem Spritzkanal 54 in Richtung der Spritzennadel 30 unterbrochen. Wird nun der mit einem Betätigungshebel 57 als Öffnungsorgan versehene Ventilstift 55 um 90° wie mit strichlierten Linien des Betätigungshebels 57 und des Ventilstiftes 55 in Fig. 6 bzw. 7 dargestellt, so kann die Injektionslösung im Bereich der Abflachungen 56 den Ventilstift 55 ungehindert passieren und in die Spritzennadel 30 bzw. deren Spritzenkanal eintreten. Wie ein Vergleich der Fig. 6 und 7 weiters zeigt, ist jedoch eine Betätigung des Betätigungshebels 57 nur dann möglich, wenn die Nadelschutzkappe 25 − wie in Fig. 7 gezeigt − entfernt ist. Somit ist auch hier ein ausreichender Originalitätsverschluss gegeben bzw. kann eine Betätigung oder Öffnung der durch den Ventilstift 55 gebildeten Verschlussvorrichtung 58 erst nach Öffnen des Originalitätsverschlusses erfolgen. Somit ist auch die Sterilität und der Einmalgebrauch derartiger Injektionsspritzen sichergestellt.

In Fig. 8 ist eine andere Ausführungsvariante eines Öffnungsorganes 59 für eine durch eine Membran 38 gebildete Verschlussvorrichtung 60 gezeigt. Der Originalitätsverschluss bzw. die Öffnungsvorrichtung einer Nadelschutzkappe 25 ist wieder entsprechend einer der Ausführungsformen gemäss den Fig. 1 bis 7 ausgebildet. Das Öffnungsorgan 59 für die Membran 38 wird im vorliegenden Ausführungsbeispiel jedoch dadurch gebildet, dass einem Nadelträger 61 ein relativ zu diesem verstellbarer Stützkörperteil 62, in welchem die Spritzennadel 30 eingespritzt ist, zugeordnet ist. Dieser Stützkörperteil 62 weist einen rohrförmigen, sich in Richtung der Auslassöffnung 36 des Ansatzstückes 19 vorspringenden rohrförmigen Nadelteil 63 auf, der aus dem gleichen Kunststoff wie der Stützkörperteil 62 oder aber auch durch einen in den Stützkörperteil 62 eingespritzten Metallteil oder aus dem gleichen Material, beispielsweise Metal wie der Stützkörperteil, hergestellt sein kann. Dieser Nadelteil 63 ist an seiner der Auslassöffnung 36 zugewandten Stirnseite 64 mit einer schrägen Stirnfläche versehen. Wird nun die Nadelschutzkappe 25 durch Aufbrechen der Schwächungslinie 23 abgetrennt und entfernt, so kann der Stützkörperteil 62 durch eine kombinierte, durch Pfeile 65 und 66 angedeutete Längs- und Drehbewegung in Richtung der Membran 38 vorgeschoben werden, so dass die Stirnseite 64 die Membran 38 durchstösst. Je nach der Art der Membran kann diese dabei abgetrennt oder nur durchstossen werden. Somit wird eine direkte Verbindung zwischen dem Spritzennadelkanal 39 der Spritzennadel 30 und der Auslassöffnung 36 hergestellt. Wird überdies der Nadelteil 63 oder ein entsprechendes Ansatzstück dichtend im Nadelträger 61 geführt, so ist auch durch die Öffnungsbewegung und Längsbewegung des Stützkörperteiles 62 in Richtung des Pfeiles 66 die Sterilität absolut gewahrt.

Es sei lediglich der Ordnung halber darauf hingewiesen, dass selbstverständlich auch möglich ist, anstelle der kombinierten Längs- und Drehbewegung gemäss den Pfeilen 65 und 66 das Durchstossen der Membran 38 nur durch eine Längsbewegung in Richtung des Pfeiles 66 oder bei entsprechender Gestaltung, beispielsweise einer schräg zur Längsrichtung der Auslassöffnung 36 erfolgten Anordnung der Membran 38 nur durch eine Drehung des Stützkörperteiles 62 diese Membran 38 zu öffnen.

In Abänderung der dargestellten Ausführungsform in Fig. 8 ist es aber auch möglich, anstelle des rohrförmigen Nadelteils 63 eine volle Nadel in der Mitte des Spritzkanals des Nadelträgers 61 anzuordnen, mit der die Membran 38 durchstossen wird, so dass durch die derart hergestellte Öffnung das Injektionsmedium in den Spritzennadelkanal 39 eintreten kann.

Die Ausbildung und Anordnung der Verschlussvorrichtung und des dieser zugeordneten Öffnungsorgans ist selbstverständlich völlig unabhängig von der Ausbildung der Öffnungsvorrichtung der Nadelschutzvorrichtung und umgekehrt. Sinnvollerweise ergeben sich jedoch weitere zusätzliche Vorteile und Effekte durch die Kombination dieser beiden Lösungsvarianten, wobei jedoch auch die Anordnung von zwei beispielsweise durch Schwächungslinien gebildeten Öffnungsvorrichtungen im Zuge einer Nadelschutzkappe 25 für sich eine eigenständige Erfindung darstellen kann.

**Patentansprüche**

1. Injektionsspritze (17) mit einem Spritzenkörper (1, 18) und einem in diesen eingesetzten, eine Spritzennadel (4, 30) aufnehmenden Nadelträger (5, 26, 50, 61) und einer mit dem Nadelträger kraft- und bzw. oder formschlüssig verbundenen, mit einem Kupplungsteil des Spritzenkörpers zumindest teilweise formschlüssig kuppelbaren Haltevorrichtung (11, 21), einer die Spritzennadel (4, 30) umhüllenden und sich bis in den Bereich des Nadelträgers (5, 26, 50) erstreckenden Nadelschutzkappe (12, 25) und einer zwischen dem Nadelträger (5, 26, 50, 61) und dem der Nadelspitze zugeordneten Ende der Nadelschutzkappe (12, 25) angeordneten, insbesondere durch eine Schwächungslinie (14, 23, 46) gebildeten Öffnungsvorrichtung, dadurch gekennzeichnet, dass zwischen dem Spritzenkörper (18) und dem von diesem abgewendeten Ende der Spritzennadel (30) eine durch eine Membran (38) gebildete Verschlussvorrichtung (58, 60) für einen Spritzkanal (40) und bzw. oder einen Spritzennadelkanal (39) angeordnet ist, welcher ein innerhalb der Nadelschutzkappe (12, 25) angeordnetes Öffnungsorgan zugeordnet ist.

2. Injektionsspritze nach Anspruch 1, dadurch gekennzeichnet, dass der Membran (38) als Öffnungsorgan eine im Spritzennadelkanal (39) und Spritzkanal (40) angeordnete Nadel (41) mit einem ausserhalb der Spritzennadelspitze liegenden Betätigungsorgan (42, 44) zugeordnet ist.

3. Injektionsspritze nach einem der Ansprüche 1 bis 7, dadurch gekennzeichnet, dass das Öffnungsorgan aus zumindest zwei in Längsrichtung des Spritzennadelkanals (39) verstell- und bzw. oder verdrehbaren Teilen des Nadelträgers (50) gebildet ist und die Verschlussvorrichtung (60) durch eine im Verstellbereich der beiden Teile angeordnete flüssigkeitsdichte Membran (38) oder dgl. gebildet ist.

4. Injektionsspritze nach Anspruch 1, dadurch gekennzeichnet, dass der Führungsteil (29) in einem gegenüber dem Ringflansch (6, 28) vorspringenden Bereich zylinderförmig, vorzugsweise als Kegelstumpf ausgebildet ist, der in etwa einen gleichen Durchmesser (35) wie die Auslassöffnung (36) des Spritzenkörpers (18) aufweist und dessen Mantelumfangsfläche als Dichtfläche ausgebildet ist.

5. Injektionsspritze nach Anspruch 1, dadurch gekennzeichnet, dass ein Ringflansch (6, 28) des die Verschlussvorrichtung aufnehmenden Nadelträgers (5, 26, 50, 61) unter Zwischenschaltung einer Dichtung (32) mit das Ansatzstück übergreifenden klauenförmigen Vorsprüngen (22) bzw. einem Ringbord der Haltevorrichtung (11, 21) bzw. eines Basisteiles der Nadelschutzkappe, welche in einer nutförmigen Vertiefung (20) bzw. einer Ringnut des Ansatzstückes (2, 19) verrastbar sind, in Anlage an die Stirnseite (33) des Ansatzstückes (2, 19) gehalten ist.

6. Injektionsspritze nach Anspruch 1, dadurch gekennzeichnet, dass eine der Nadelspitze zugewandte Ringfläche des Ringflansches (6, 28) des Nadelträgers (5, 26, 50, 61) mit der Haltevorrichtung (11, 21) bzw. dem Basisteil der Nadelschutzvorrichtung verklebt oder verschweisst ist.

7. Injektionsspritze nach Anspruch 1, dadurch gekennzeichnet, dass ein Konvergenzwinkel der Konizität der Haltevorrichtung (11, 21) kleiner ist als ein Konvergenzwinkel des gleichfalls einen konisch ausgebildeten Mantel aufweisenden Ansatzstückes (2, 19).

8. Injektionsspritze nach einem der Ansprüche 1 bis 7, dadurch gekennzeichnet, dass der Nadelschutzkappe (12, 25) in ihrem der Nadelspitze zugewandten Endbereich eine weitere Öffnungsvorrichtung (45) zugeordnet ist, wobei im Bereich zwischen dieser Öffnungsvorrichtung (45) und dem der Nadelspitze zugewandten Ende der Nadelschutzkappe (12, 25) ein Betätigungsorgan (44) der Verschlussvorrichtung angeordnet ist.

**Claims**

1. Injection syringe (17) comprising a syringe body (1, 18) and a needle carrier (5, 26, 50, 61) inserted therein and receiving a syringe needle (4, 30), and a holding device (11, 21) connected to the needle carrier in force locked and/or shape locked manner and liable to be coupled at least partially in shape locked manner to a coupling element of the syringe body, a needle protector cap (12, 25) sheathing the syringe needle (4, 30) and extending into the area of the needle carrier (5, 26, 50) and an opening device formed in particular by a weakening line (14, 23, 46) and situated between the needle carrier (5, 26, 50, 61) and the extremity associated with the needle tip of the needle protector cap (12, 25), characterised in that a closure device (58, 60) for a syringe passage (40) and/or a syringe needle passage (39) and formed by a diaphragm (38) is situated between the syringe body (18) and the extremity of the syringe needle (30) facing away from the former, which has allocated to it an opening element situated within the needle protector cap (12, 25).

2. Injection syringe according to claim 1, characterised in that, as an opening element, a needle (41) having an actuator element (42, 44) situated beyond the syringe needle tip, and placed in the syringe needle passage (39) and syringe passage (40), is associated with the diaphragm (38).

3. Injection syringe according to one of the claims 1 to 7, characterised in that the opening element is formed by at least two parts of the needle carrier (50) displaceable and/or twistable in the longitudinal direction of the syringe needle passage (39), and the closure device (60) is formed by a fluid-tight diaphragm (38) or the like which is situated in the range of displacement of the two parts.

4. Injection syringe according to claim 1, characterised in that the locating element (29) is constructed in an area projecting with respect to the annular flange (6, 28) in cylindrical shape and preferably as a cone frustum, which has approximately the same diameter (35) as the outlet opening (36) of the syringe body (18) and whereof the peripheral jacket surface is formed as a sealing surface.

5. Injection syringe according .to claim 1, characterised in that an annular flange (6, 28) of the needle carrier (5, 26, 50, 61) receiving the closure device is held in contact under interpositioning of a seal (32) by means of claw-like projections (22) or of an annular rim of the holding device (11, 21) or a base portion of the needle protector cap, which may engage in a groove-shaped depression (20) or an annular groove of the attachment member (2, 19), against the end side (33) of the attachment member (2, 19).

6. Injection syringe according to claim 1, characterised in that an annular surface facing towards the needle tip of the annular flange (6, 28) of the needle carrier (5, 26, 50, 61) is bonded or welded to the holding device (11, 21) or the base element of the needle protection device.

7. Injection syringe according to claim 1, characterised in that a convergency angle of the taper of the holding device (11, 21) is smaller than a convergency angle of the attachment member (2, 19) which equally has a conically formed jacket.

8. Injection syringe according to one of the claims 1 to 7, characterised in that another opening device (45) is associated with the needle protector cap (12, 25) in its terminal portion facing towards the needle tip, an actuator element (44) for the closure device being situated in the area between this opening device (45) and the extremity facing towards the needle tip of the needle protector cap (12, 25).

## Revendications

1. Seringue d'injection (17) avec un corps de seringue (1, 18) et un support d'aiguille (5, 26, 50, 61) inséré dans celui-ci et recevant une aiguille de seringue (4, 30), et un dispositif de maintien (11, 21) relié au support d'aiguille par assemblage par force et/ou par assemblage de forme et couplage au moins partiellement par assemblage de forme avec une partie de couplage du corps de seringue, un capuchon de protection de l'aiguille (12, 25) entourant l'aiguille de seringue (4, 30) et s'étendant jusque dans le domaine du support d'aiguille (5, 26, 50) et un dispositif d'ouverture disposé entre le support d'aiguille (5, 26, 50, 61) et l'extrémité du capuchon de protection de l'aiguille (12, 25) associée à la pointe de l'aiguille, et formé en particulier par une ligne d'affaiblissement (14, 23, 46), caractérisée en ce qu'entre le corps de seringue (18) et l'extrémité éloignée de celui-ci de l'aiguille de la seringue (30) est disposé un dispositif de fermeture (58, 60) formé par une membrane (38) pour un canal d'injection (40) et/ou un canal d'aiguille de seringue (39), auquel est associé un organe d'ouverture disposé à l'intérieur du capuchon de protection de l'aiguille (12, 25).

2. Seringue d'injection selon la revendication 1, caractérisée en ce qu'une aiguille (41) disposée dans le canal de l'aiguille de seringue (39) et dans le canal d'injection (40) est associée à la membrane (38) en tant qu'organe d'ouverture avec un organe d'actionnement (42, 44) disposé à l'extérieur de la pointe de l'aiguille de seringue.

3. Seringue d'injection selon la revendication 1, caractérisée en ce que l'organe d'ouverture est formé par au moins deux parties du support d'aiguille (50) déplaçables et/ou susceptibles de tourner le long du canal de l'aiguille de seringue (39) et en ce que le dispositif de fermeture (60) est formé par une membrane (38) ou analogue étanche au liquide disposée dans le domaine de déplacement des deux parties.

4. Seringue d'injection selon la revendication 1, caractérisée en ce que la partie de guidage (29) est cylindrique, de préférence sous la forme d'un tronc de cône dans un domaine en saillie par rapport à la bride annulaire (6, 28), lequel tronc de cône présente un diamètre (35) à peu près égal à celui de l'ouverture de sortie (36) du corps de seringue (18) et dont la surface périphérique d'enveloppe est sous la forme d'une surface d'étanchéité.

5. Seringue d'injection selon la revendication 1, caractérisée en ce qu'une bride annulaire (6, 28) du support d'aiguille (5, 26, 50, 61) recevant le dispositif de fermeture est maintenue en position contre la face frontale (33) de l'embout (2, 19) avec insertion d'un joint (32) par des saillies (22) en forme de griffes enserrant l'embout ou un bord annulaire du dispositif de maintien (11, 21) ou d'une partie de base du capuchon de protection de l'aiguille, qui peuvent s'encliqueter dans un évidement (20) en forme de rainure ou dans une rainure annulaire de l'embout (2, 19).

6. Seringue d'injection selon la revendication 1, caractérisée en ce qu'une surface annulaire, tournée vers la pointe de l'aiguille, de la bride annulaire (6, 28) du support d'aiguille (5, 26, 50, 61) est collée ou soudée au dispositif de maintien (11, 21) ou à la partie de base du dispositif de protection de l'aiguille.

7. Seringue d'injection selon la revendication 1, caractérisée en ce qu'un angle de convergence de la conicité du dispositif de maintien (11, 21) est plus petit qu'un angle de convergence de l'embout (2, 19) présentant de même une enveloppe de forme conique.

8. Seringue d'injection selon l'une des revendications 1 à 7, caractérisée en ce qu'un autre dispositif d'ouverture (45) est associé au capuchon de protection de l'aiguille (12, 25) dans son domaine d'extrémité tourné vers la pointe de l'aiguille, un organe d'actionnement (44) du dispositif de fermeture étant disposé dans le domaine entre ce dispositif d'ouverture (45) et l'extrémité du capuchon de protection de l'aiguille (12, 25) tournée vers la pointe de l'aiguille.

**Fig.1**

**Fig.2**

**Fig.3**

EP 0 235 139 B1

**Fig.4**

**Fig.5**

EP 0 235 139 B1

Fig.6

Fig.7

Fig.8